# EUROPEAN PATENT SPECIFICATION

(11) **EP 0 938 925 B1**
(45) Date of publication and mention of the grant of the patent: **31.08.2005**
(21) Application number: 99103446.3
(22) Date of filing: 23.02.1999
(51) Int. Cl.: B01J 20/24, B01J 20/26, B01J 20/18, D21H 17/68, D21H 11/16, B32B 19/00, C08K 9/08, D01F 1/10, C09D 5/00

(54) **Composition containing a zeolite-cellulose composite and product made therefrom**
Zusammensetzung, die ein Zeolith-Cellulose-Verbundmaterial enthält, und damit hergestelltes Produkt
Composition contenant un materiau composite zéolithe-cellulose et produit obtenu à partir de celle-ci

(30) Priority: 25.02.1998 JP 4353498; 25.02.1998 JP 4353998; 25.02.1998 JP 4363298
(43) Date of publication of application: 01.09.1999
(73) Proprietor: Rengo Co., Ltd., Osaka 553-0007 (JP)
(72) Inventor: Sugiyama, Kouju, c/o Rengo Co., Ltd., Central Lab., Osaka-shi, Osaka 553-0007 (JP); Nakano, Maki, c/o Rengo Co., Ltd., Central Lab., Osaka-shi, Osaka 553-0007 (JP); Utsunomiya, Takaaki,c/o Rengo Co.Ltd.,Central Lab., Osaka-shi, Osaka 553-0007 (JP); Fujimoto, Yoshinobu,c/o Rengo Co.Ltd.,Central Lab., Osaka-shi, Osaka 553-0007 (JP)
(74) Representative: von Kreisler, Alek

(56) References cited:
- EP-A- 0 457 235
- EP-A- 0 826 822
- WO-A-92/16291
- JP-A- 5 017 124
- JP-A- 5 106 199
- US-A- 3 099 570
- US-A- 4 775 585
- DATABASE WPI Section Ch, Week 9751 Derwent Publications Ltd., London, GB; Class E33, AN 97-554978 XP002103624 & JP 09 268500 A (OJI PAPER CO) , 14 October 1997
- PATENT ABSTRACTS OF JAPAN vol. 010, no. 031 (C-327), 6 February 1986 & JP 60 181370 A (KANEBO KK), 17 September 1985
- PATENT ABSTRACTS OF JAPAN vol. 014, no. 423 (C-0757), 12 September 1990 & JP 02 160915 A (KANEBO LTD), 20 June 1990
- DATABASE WPI Section Ch, Week 8941 Derwent Publications Ltd., London, GB; Class A92, AN 89-297758 XP002103625 & JP 01 221242 A (DAI NIPPON PRINTING KK) , 4 September 1989
- DATABASE WPI Section Ch, Week 9223 Derwent Publications Ltd., London, GB; Class A11, AN 92-188655 XP002103626 & JP 04 122743 A (KANEBO LTD) , 23 April 1992
- DATABASE WPI Section Ch, Week 7949 Derwent Publications Ltd., London, GB; Class A82, AN 79-88508B XP002103627 & JP 54 139640 A (TOYO SODA MFG CO LTD) , 30 October 1979

## Description

### TECHNICAL FIELD OF THE INVENTION

The present invention relates to a composition comprising a function improver (B) and an inorganic porous zeolite crystals-cellulose composite (A), which is capable of removing bad odor, adsorbing gas and the like, and which is superior in antibacterial property, noncombustibility, heat insulating property, strength and other properties, wherein the cellulose contains inorganic porous zeolite crystals in its inner matrix, and to a product comprising the above-mentioned components (A) and (B).

### BACKGROUND OF THE INVENTION

A material obtained by carrying an inorganic compound, such as zeolite on a hydrophilic macromolecule substrate of cellulose substrate, such as paper and the like, to impart various functions has been known. Such material can be used for various applications since it can remove bad odor, adsorb gas and the like, and shows antibacterial property, noncombustibility and heat insulating property.

Inasmuch as the above-mentioned material is expected to be useful in vanous applications, one having various high functions, such as higher strength and the like, has been desired to meet the requests of actual end users.

JP-A-60181370 relates to artificial leather suitable as shoe leather having high antimicrobial activity and durability for a long time that consists of a resin and a cellulosic material containing zeolite solid particles containing a metal ion having a sterilizing effect. The solid zeolite particles are located in the inside and on the outside of the cellulose.

JP-A-5017124 discloses a composite containing zeolite located in a cellulose network, i.e., in its inner matrix. The composite is prepared by contacting the cellulose with a gel containing zeolite precursor compound thereby forming zeolite within the cellulose ntework.

JP-A-5106199 discloses a fiber containing inorganic silicon compound supported in its interior. In addition salts of Cn, Ag or Zn can be incorporated in the fiber by treating it with a respective aqueous solution.

US-A-3099570 discloses a filler composed of fibers which are permeated and coated with aluminum silicate. The filler is used for paper making, i.e., it is combined with further "function improvers".

It is therefore an object of the present invention to provide a material fulfilling such requests, which is an inorganic porous crystals-hydrophilic macromolecule composite having improved functions besides the functions of removing bad odor, adsorbing gas and the like, antibacterial property, noncombustibility, heat insulating property and the like, wherein the hydrophilic macromolecule contains inorganic porous crystals in its inner matrix.

### SUMMARY OF THE INVENTION

Such object can be achieved by the present invention described in the following.

The present invention provides the following.
1. A composition comprising
   (A) an inorganic porous zeolite crystals-cellulose composite wherein
      (i) the cellulose is natural cellulose which swells with water, and
      (ii) a part or the entirety of the inorganic porous zeolite crystals is contained in the inner matrix of the cellulose, the inner matrix being the inside of a substance constituting the cellulose not including fine pores and lumen present on the surface of the cell wall or in the cell wall of the cellulose, and
   (B) a function improver which is a carrier capable of solidifying and being a polyolefin.
2. The composition of point 1, wherein the inorganic porous crystals hold at least one metal selected from the group consisting of silver, copper, zinc, iron, nickel, cobalt, palladium and platinum.
3. The composition of point 1, wherein the natural cellulose is at least one member selected from the group consisting of wood powder, pulp, cotton, hemp and kenaf.
4. A product obtainable by solidifying the composition as defined in any of points 1 to 3.
5. The product of point 4, which is a laminate comprising a layer made from composite (A) and a function improving substrate (B2) being a polyolefin.
6. The product of point 4 being selected from a textile, nonwoven fabric or paper comprising a fiber made from composite (A) and a function improving fiber (B1) being a polyolefin.

### BRIEF DESCRIPTION OF THE DRAWINGS

Fig. 1 shows one embodiment of the laminate of the present invention, wherein the inorganic porous crystals-hydrophilic macromolecule composite (A) is an inorganic porous crystals-pulp composite. In the Figure, 1 is a substrate made from a fiber and 2 is a layer made from a fiber of an inorganic porous crystals-pulp composite.
Fig. 2 shows another embodiment of the laminate of the present invention, wherein the inorganic porous crystals-hydrophilic macromolecule composite (A) is an inorganic porous zeolite crystals-pulp composite. In the Figure, 1' is a film-like substrate and 2 is a layer made from a fiber of the inorganic porous zeolite crystals-pulp composite.
Fig. 3 shows a film-like laminate of the present invention, wherein 2' is a film-like inorganic porous zeolite crystals-cellulose composite.
Fig. 4 shows a tube-like embodiment of the laminate of the present invention, wherein 2" is a tube-like layer made from an inorganic porous zeolite crystals-cellulose composite fiber.
Fig. 5 shows an embodiment of a sheet of the laminate of the present invention.
Fig. 6 is a graph showing nitrogen dioxide removing capability of each test piece in Experiment 1.
Fig. 7 is a graph showing ammonia removing capability of each test piece in Experiment 1.
Fig. 8 is a graph showing ammonia removing capability of each test piece in Experiment 2.

### DETAILED DESCRIPTION OF THE INVENTION

### Inorganic porous crystals

The inorganic porous zeolite crystal of the inorganic porous-zeoli cellulose composite (A) to be used in the present invention is, for example, inorganic ion exchange zeolite crystal having ion exchange capability or adsorber zeolite crystal having adsorption capability in the porous part. It is free of particular limitation as long as it does not dissolve, decompose or disintegrate a hydrophilic macromolecule.

Zeolite is used in view of its widest uses, with particular preference given to 4A zeolite [Na₁₂Si₁₂Al₁₂O₄₈ · 27H₂O] for comparatively easy synthesis.

### Cellulose

The cellulose of the inorganic porous zeolite crystals-cellulose composite (A) to be used in the present invention is subject to no particular limitation as long as it is natural cellulose that swells with water. Examples thereof include natural cellulose such as pulp and kenaf, regenerated cellulose (e.g., cellophane, cellulose beads, rayon, cellulose sponge and the like), cotton, bacterial cellulose, wood powder and the like.

Of these, pulp and regenerated cellulose are preferably used in view of the mode of actual use, price and easy handling.

### Inorganic porous zeolite crystals-cellulose composite (A)

The above-mentioned hydrophilic macromolecule holds inorganic porous zeolite crystals in its inner matrix and forms the inorganic porous zeolite crystals-cellulose composite to be used in the present invention. As used herein, by "in its inner matrix of cellulose" is meant, the inside of a substance constituting the cellulose. Fine pores and lumen present on the surface of the cell wall or in the cell wall of the cellulose are not included.

Having inorganic porous crystals in its inner matrix of cellulose means that a part or the entirety of the inorganic porous crystals is present in its inner matrix of the cellulose.

The inorganic porous zeolite crystals-cellulose composite (A) can be prepared as in the following. In case of a zeolite -cellulose composite wherein the inorganic porous crystal is zeolite for example, the method disclosed in Japanese Patent Unexamined Publication No. 8-245538 is used. To be specific, a cellulose substrate is immersed in a 1.0 - 100 mmol/1 aqueous solution of silicon compound for 10 min to 2 hr and then in an aqueous solution of a mixture of 1.0 - 1000 mmol/1 of an aluminum compound and 10 - 5000 mmol/1 of a basic substance at 20-90°C for 2 hr to 20 days.

When the hydrophilic macromolecule is pulp, cellophane or rayon, an inorganic porous crystals-pulp composite, an inorganic porous crystals-cellophane composite, and an inorganic porous crystals-rayon composite can be obtained by a method similar to the above-mentioned method.

The proportion of the inorganic porous zeolite crystals and hydrophilic macromolecule in the inorganic porous zeolite crystals-cellulose composite (A) is not particularly limited. The cellulose is preferably contained in (A) in a proportion of 1.0 - 70.0% by weight, particularly preferably 10.0 - 50.0% by weight.

### Metal-holding inorganic porous zeolite crystals-cellulose composite

A metal-holding inorganic porous zeolite crystals-cellulose composite can be obtained by immersing the above-mentioned inorganic porous zeolite crystals- cellulose composite in an aqueous solution of a metal salt having a catalytic function. The metal to be used includes, for example, silver, copper, zinc, iron, nickel, cobalt, palladium, platinum and the like, which may be used in combination. While the concentration of the aqueous solution of a metal salt is not particularly limited, it is preferably 1.0 - 100 mmol/l, and the temperature and time of immersion are not particularly limited, either.

Inasmuch as the cellulose permeates an aqueous solution, a metal can be held by the whole inorganic porous zeolite crystals in the inner matrix of the cellulose.

While the content of the metal in the metal-holding inorganic porous zeolite crystals- cellulose composite is not particularly limited, it is preferably 0.001-10.0 wt%, particularly preferably 0.01 - 1.0 wt%.

For example, the inorganic porous zeolite crystals-cellulose composite holding silver, copper or zinc shows antibacterial property, and the inorganic porous zeolite crystals-cellulose composite holding palladium or platinum adsorbs ethylene. Thus, they are effective in retention of freshness of vegetables and fruits. The inorganic porous zeolite crystals-cellulose composite holding silver or copper can adsorb or decompose hydrogen sulfide, so that it has an anticorrosion effect on a metal and deodorizing effect, as well as odor preventive effect based on absorption or decomposition of ammonium. Further, the inorganic porous zeolite crystals-cellulose composite holding silver can adsorb and decompose methyl mercaptane, so that it is effective for odor prevention. In this case, since can fully permeate gases, the entire metal-holding inorganic porous crystals in the inner matrix of the can be utilized to adsorb or decompose gases.

### Function improver (B)

The composition of the present invention contains a member (B) for improving the functions such as strength and the like of a product obtained from this composition. The function improver (B) is a carrier capable of solidifying which is a polyolefin. This carrier is not particularly limited as long as it can be solidified by some means after adding, to this carrier, an inorganic porous zeolite crystals-cellulose composite (A) wherein the cellulose contains inorganic porous crystals in its inner matrix. The means of solidifying may be, for example, heating, cooling, compression, chemical reaction (e.g., oxidation, enzyme reaction and the like), and the like. The degree of solidification may be such that it affords at least the production of a product, wherein said product may be one having bendability or may be deformable.

The carrier improves functions such as strength upon solidification that allows it to adhere to the composite (A).

Examples of such carrier include resin. Examples of said resin include synthetic resin.

The synthetic resin is, polyolefin (e.g., polyethylene, polypropylene and the like). In addition, butadiene rubber can be also used. Preferred is polyethylene in view of price, low boiling point and general applicability.

The mode of the carrier using a resin include, for example, a liquid (e.g., paint and the like), flexible one like rubber, board, sheet, string, net, cushion.

### Composition

The composition of the present invention can be produced by adding, to a function improver (B), an inorganic porous zeolite crystals-cellulose composite (A) wherein the cellulose contains inorganic porous crystals in its inner matrix. To obtain a product, a flowable function improver, such as a paint, is applied to a composite (A) to be coated, which is followed by forming a film to give a product. A paint containing an acrylic resin is an emulsion, and evaporation of water therefrom results in a film. A paint containing an epoxy resin solidifies upon chemical reaction to give a product. The thickness of the product is 10-500 µm, preferably about 20-100 µm. The thickness of the product solidified using an inorganic hardener is about 1-10 cm. When the product is a sphere or ellipse, it has a long diameter of 0.1-5 cm, preferably 0.5-2 cm. A spherical product solidified using an inorganic hardener has the same size as above.

The shape of the composition after solidification is not particularly limited, and may be a sphere, cube, column, plate and the like.

The product obtained by solidification of the composition of the present invention and production method of the composition (liquid) of the present invention are explained in detail by referring to specific examples.

### 1. Production method of product obtained by solidification

### a. Plastic

A zeolite-cellulose bead composite is added to a polyethylene master batch and the mixture is kneaded in a kneader set to 130°C. The kneaded product is extruded through a mold and cooled to solidify.

### a. Paint

To a paint is added zeolite-cellulose bead composite, followed by kneading.

The proportion of the inorganic porous zeolite crystals-cellulose composite (A) to be added is about 1-60 parts by weight per 100 parts by weight of the function improver (B). Where necessary, auxiliary agents such as surfactant, plasticizer, antioxidant, dispersant, precipitation preventive, wood filler, oil stain and the like may be added besides the function improver (B).

### Textile,nonwoven fabric and paper

Examples of the product of the present invention include a fiber (hereinafter also referred to as inorganic porous zeolite crystals-cellulose composite fiber (A)) made from an inorganic porous zeolite crystals-cellulose composite (A) wherein the cellulose contains inorganic porous zeolite crystals in its inner matrix, and textile, nonwoven fabric and paper containing, as function improving fiber (B1), a fiber other than fiber (A), which is a polyolefin fiber.

### Function improving fiber (B1)

The function improving fiber (B1) is a polyolefin fiber other than composite fiber (A) and is free of inorganic porous zeolite crystals. It has an appropriate strength (e.g., one having a strength measured according to JIS L1069 "test method of tensile strength of fiber" of about 4-1500 gf/d, preferably about 20-500 gf/d). The above-mentioned chemical fiber may be made from a copolymer.

The fineness, sectional shape, presence or absence of various polymer stabilizers, basic weight, density of the function improving fiber (B 1) are not particularly limited.

### Textile, nonwoven fabric or paper

The textile, nonwoven fabric and paper of the present invention can be produced from an inorganic porous zeolite crystals-cellulose composite fiber (A), and a function improving polyolefin fiber (B1) according to a method known in the field of art. This textile includes mixed spinning textile, union cloth depending on the kind of the yarns used for the warp and the woof of the textile. In view of the strength, a mixed spinning textile is preferable. The density of the warp and the woof constituting the textile are not particularly limited.

The method for producing the nonwoven fabric include dry production method, wet production method. In view of simplification of the production method and the kind of starting material, a dry production method is preferable. For example, when a thermoplastic resin such as polyethylene is employed, a dry production method is preferable.

Paper can be produced by a conventional paper making method (wet method and the like). When a fiber (B1) is hydrophobic fiber such as polyethylene and the hydrophobic fiber is used in an amount smaller than that of the pulp in the fiber (A), a wet method is preferable. In the latter case, each ingredient needs to be mixed well, and an additive for this end, such as surfactant can be added as necessary.

The proportion of the inorganic porous zeolite crystals-cellulose composite fiber (A) and function improving fiber (B1) to be used is not particularly limited, and (A) : (B) is preferably 10 : 90-90 : 10 by weight, particularly preferably 40 : 60-60 : 40 by weight.

The thickness of the textile, nonwoven fabric and paper of the present invention is 20-500 µm, preferably 30-100 µm.

The textile, nonwoven fabric and paper of the present invention can contain, where necessary, additives such as polyacrylamide, starch and the like for enhanced paper strength, inorganic pigment for increased whiteness, and active charcoal, silica gel, hydrophobic zeolite, titanium dioxide and the like for improved multifunctions, and the like, besides the inorganic porous crystals-hydrophilic macromolecule composite fiber (A) and function improving fiber (B1).

### Laminate

A different product of the present invention includes a laminate comprising a layer made from the inorganic porous zeolite crystals-cellulose composite (A), wherein the hydrophilic macromolecule contains inorganic porous crystals in its inner matrix, and a function improving substrate (B2), which is a polyolefin.

### Substrate (B2)

The function improving substrate (B2) to be used in the present invention is exemplified by one having an appropriate strength (e.g., a strength measured according to JIS P8113 "test method of tensile strength of paper and paper board" of 300-1000 gf, preferably 500-750 gf, or a strength measured according to JIS L1069 "test method of tensile strength of fiber" of 4-1500 gf/d, preferably 20-500 gf/d) and other characteristics such as a good touch, weatherability, water resistance, heat insulating property, electromagnetic resistance and the like.

Specific examples include a sheet and the like of knit fabric, textile, nonwoven fabric and the like made from at least one fiber selected from polyolefin fiber. The above-mentioned polyolefin fiber may be a copolymer. The fineness, sectional shape, presence or absence of various polymer stabilizers, basic weight, density and the like of the sheet are not particularly limited.

The thickness of these sheet substrates is 1-500 µm, preferably 10-100 µm.

The substrate (B2) to be used in the present invention is exemplified by, in addition to the sheet made from the above-mentioned fiber, a plastic film or sheet (hereinafter also referred to as a plastic film) made from a resin such as polyolefin. When high weatherability and water resistance are desired, the use of a sheet or film made from a polyolefin resin such as polyethylene, polypropylene, polystyrene, polyethylene terephthalate is preferable.

While the plastic film may be drawn or not drawn, a drawn one is preferable in view of strength. The method of drawing and drawing ratio are not particularly limited. This plastic film may be a foamed plastic film in view of heat insulating property and improved buffering property.

A foamed plastic film is produced by mixing a gas or a liquid capable of gasifying into a thermoplastic resin under pressurization, and heating or bringing to normal pressure, thereby to cause foaming, mixing a degradable foaming agent, heating to cause decomposition, thereby to cause foaming, or foaming with a gas generated during polymerization reaction, and other method. The expansion ratio is not particularly limited. Examples of foamed plastic include polystyrene foam, and the like.

The thickness of the plastic film is 10-500 µm, preferably 20-100 µm.

The substrate (B2) to be used in the present invention contains at least one member selected from the sheets of the above-mentioned knit fabric, textile and nonwoven fabric and the like, and plastic film.

A substrate is produced from two different kinds of materials, for example, polyethylene film and metal foil, or sheets of knit fabric, textile, nonwoven fabric and the like and a thermoplastic resin film, by superimposing the two different materials and passing them through a heat roll for thermal welding and other method. A method comprising forming an adhesive layer on a board made from plaster and adhering a polyurethane foam thereto may be used.

The thickness of the layer composed of an inorganic porous zeolite crystals-cellulose composite varies depending on the purpose of use, which is preferably 10-500 µm, and more preferably 20-100 µm.

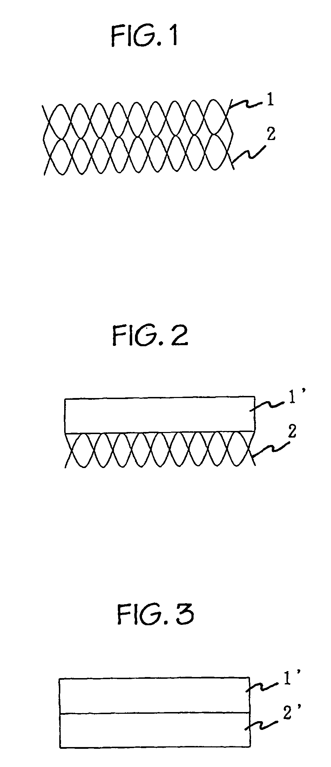

A preferable embodiment of the laminate of the present invention wherein the inorganic porous zeolite crystals-cellulose composite (A) is an inorganic porous zeolite crystals-pulp composite is shown in Fig. 1 and Fig. 2. In Fig. 1, 1 is a substrate, which is a collective fiber-like product and 2 is a layer made from an inorganic porous crystals-hydrophilic macromolecule composite (A), wherein the hydrophilic polymer is a collective fiber product.

In Fig. 2, 1' is a substrate, which is a film, 2 is a layer made from an inorganic porous zeolite crystals-cellulose composite (A), which is a collective fiber product.

The laminate shown in Fig. 1 and Fig. 2 can be used mainly for *shoji* paper, *fusuma* paper, wall paper, curtain, rug, carpet, tapestry and the like.

The layer composed of an inorganic porous zeolite crystals-cellulose composite (A) can take various shapes depending on the kind of the cellulose to be used. A preferable embodiment of the laminate of the present invention wherein the layer of the inorganic porous zeolite crystals-cellulose composite (A) is a film which is shown in Fig. 3. In Fig. 3, 1' is a substrate film and 2' is a layer composed of an inorganic porous zeolite crystals-cellulose composite (A), which is a film.

The laminate shown in Fig. 3 can be mainly used for freshness retaining sheet, anticorrosive sheet, insect preventive sheet, freshness retaining corrugated fibreboard, anticorrosive corrugated fibreboard, insect preventive corrugated fibreboard, fireproof corrugated fibreboard.

The thickness of the layer composed of an inorganic porous zeolite crystals-cellulose composite (A), which is a film, is about 10-500 µm, preferably 20-100 µm.

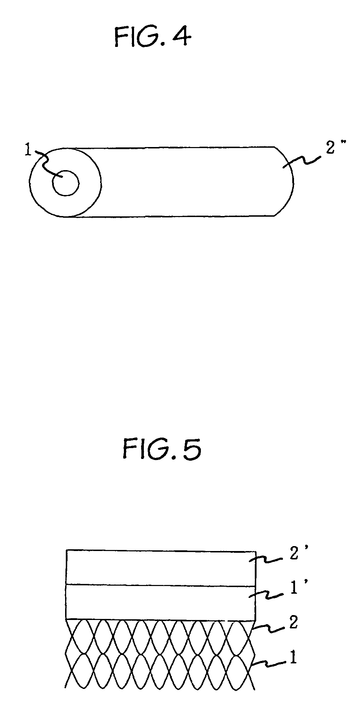

In the embodiment shown in Fig. 4, 2'' is a layer composed of an inorganic porous crystals-hydrophilic macromolecule composite (A), which is a tube and has a through-hole in the longitudinal direction, and 1 is a substrate which is filled in the through-hole. In the embodiment shown in Fig. 4, the hydrophilic macromolecule is a regenerated cellulose, and the substrate is a collective fiber. In this embodiment, the hydrophilic macromolecule is preferably a regenerated cellulose such as rayon and the like, and substrate 1 is preferably a sheet of a knit fabric, textile, nonwoven fabric and the like made from at least one member selected from plastic fiber made from polyolefin.

The laminate is mainly used for filter of air conditioner, dust bag of sweepers, filter for water tank of tropical fish, waste liquid filter and the like.

The sectional shape in the direction of the center of the through-hole is complete round, ellipse, rectangle and the like. When it is a complete round, its diameter is about 1-100 µm; when it is an ellipse, the length of the longer axis is 2-100 µm, the length of the shorter axis is 1-50 µm; sectional area thereof is generally 0.79-7900 µm², preferably 2000 µm². The longitudinal length of the composite is 1-200 µm and the length in the transverse direction is 5-1000 µm.

Fig. 5 shows an embodiment wherein a laminate of Fig. 3 is superimposed on the laminate of Fig. 1. The substrate 1 is preferably a nonwoven fabric made from a natural fiber. The layer composed of the inorganic porous zeolite crystals-cellulose composite (A) is preferably a zeolite-pulp composite layer. The substrate 1' is preferably a plastic film, particularly polyvinyl alcohol copolymer film; the layer 2' made from an inorganic porous crystals-hydrophilic macromolecule composite is preferably a zeolite-cellophane composite layer.

The thickness of this composite layer is preferably not more than 500 µm, in view of formability into a box or honeycomb, and portable property.

The laminate shown in Fig. 5 is mainly used for portable toilet for automobile, paper diaper, water purification device for survival and the like.

The layer composed of the inorganic porous zeolite crystals-cellulose composite (A) contains a volatile substance besides the above-mentioned inorganic porous crystals-hydrophilic macromolecule composite (A) to achieve high functions. Examples of the volatile substance include L-menthol, hinokitiol, fitontsid, allyl isothiocyanate, limonene and the like. These can be held by a method known in the art, such as immersion, coating, press incorporation and the like.

The laminate of the present invention can be produced by the following method. When the substrate (B2) is a sheet made from the above-mentioned fiber and the cellulose of the inorganic porous zeolite crystals-cellulose composite (A) is pulp, the substrate (B2) and the sheet of the inorganic porous zeolite crystals-cellulose composite (A) are welded to give a laminate.

When the substrate (B2) is other than the above-mentioned sheet, a layer made from the inorganic porous zeolite crystals-cellulose composite (A) is adhered to at least one surface of the substrate (B2) with an adhesive, or a layer made from the inorganic porous zeolite crystals-cellulose composite (A) is adhered while an inorganic hardener, such as cement, plaster has not solidified yet, with an adhesive to give a laminate. The thickness of the adhesive layer is 5-100 µm, preferably 10-50 µm.

In the embodiment wherein the inorganic porous crystals-hydrophilic macromolecule composite (A) has a through-hole in the longitudinal direction, this through-hole may be filled with a substrate or applied to a regeneration bath while a viscose attaches to the periphery of the substrate or other method.

The laminate of the present invention can be further laminated with, besides substrate (B2) and a layer made from the inorganic porous zeolite crystals-cellulose composite (A), for example, a sheet such as a knit fabric, textile, nonwoven fabric , which is made from a natural fiber having hydrophilicity and gas permeability. The thickness of this layer is approximately the same as that mentioned above.

When an inorganic porous zeolite crystals-cellulose composite (A) wherein the cellulose contains inorganic porous zeolite crystals in its inner matrix and a function improver (B) which is a polyolefin or function improving fiber (B1) which is a polyolefin are combined, or when a layer made from an inorganic porous zeolite crystals-cellulose composite (A) wherein the cellulose contains inorganic porous zeolite crystals in its inner matrix is laminated on a function improving substrate (B2) which is a polyolefin, the product, textile, nonwoven fabric, paper and laminate obtained from the composition of the present invention come to have high strength, in addition to the gas adsorption capability, volatile organic solvent removing capability, noncombustibility, heat insulating property, and heavy metal and radioactive element removing capability, that the inorganic porous crystals-hydrophilic macromolecule composite (A) has. It is also possible to improve a touch such as texture hydrophilicity, water repellency, anticorrosive property according to the function improvers (B), (B1) and (B2) to be used. Therefore, the inventive laminate can be used for various applications such as underwear, bath mat, sheets, gloves, pillow cover, stuffing cotton for pillow, bedding, padded sleeveless coat, cushion *shoji* paper, wall paper, clothes cover, cushion cover, bedding storage bag, insecticide sheet, pack for vacuum cleaner, filter for air conditioners, filter for air purifier, scrubbing brush for tableware, water draining garbage bag, carpet, hot carpet cover, curtain, deodorant sheet for refrigerator, special filter paper, freshness retention sheet for vegetable, meat packaging materials for freshness retention transport, wall materials, floor materials, ceiling materials, dewing absorption sheet.

Particularly, the use of a metal-holding inorganic porous zeolite crystals-cellulose composite wherein the inorganic porous zeolite crystals-cellulose composite to be used in the present invention carries a metal such as silver, copper, zinc can impart the properties of antibacterial property, odor removing capability in addition to the above-mentioned properties. Therefore, the inventive laminate can be also used for various applications such as paper diapers, diaper cover, portable toilet for automobile, insole, artificial leather, decoration of automobile, train, airplane, ship (seat, seat cover), towel, toilet seat cover.

The present invention is explained in the following by way of examples, to which the present invention is not limited.

### Production Example 1

Cellulose beads (10.0 g, average particle size 5 mm) which are molded particles of regenerated cellulose were impregnated with an aqueous solution of sodium methasilicate 9 hydrate (5.68 g/100 ml) and a mixed aqueous solution (100 ml) of sodium aluminate (4.68 g) and sodium hydroxide (10.00 g) was added, which was followed by immersion at 25°C for 10 days to give 11.2 g of zeolite-cellulose bead composite. The zeolite-cellulose bead composite had a zeolite-holding percentage of 20.8 wt%.

### Example 1 (not according to the invention)

The zeolite-cellulose bead composite obtained in Production Example 1 was added to cement for general building materials dispersed with colloidal silica and titanium dioxide, and the mixture was homogeneously kneaded. The kneaded product was placed in a mold and left standing to give a product (thickness 3.0 cm).

### Experimental Example 1 (not according to the invention)

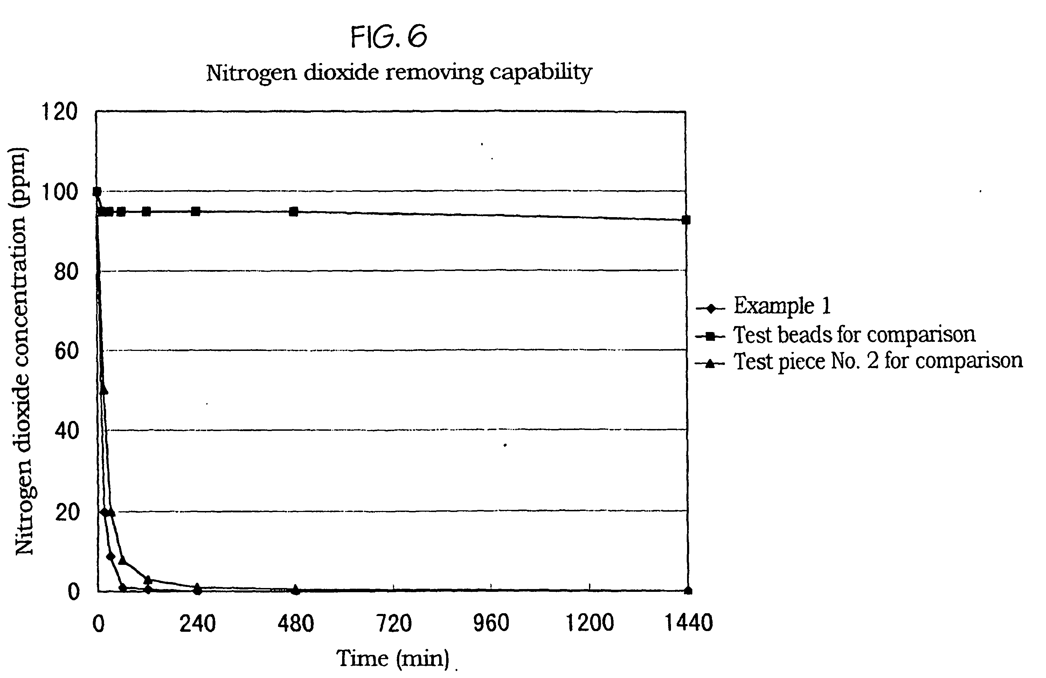

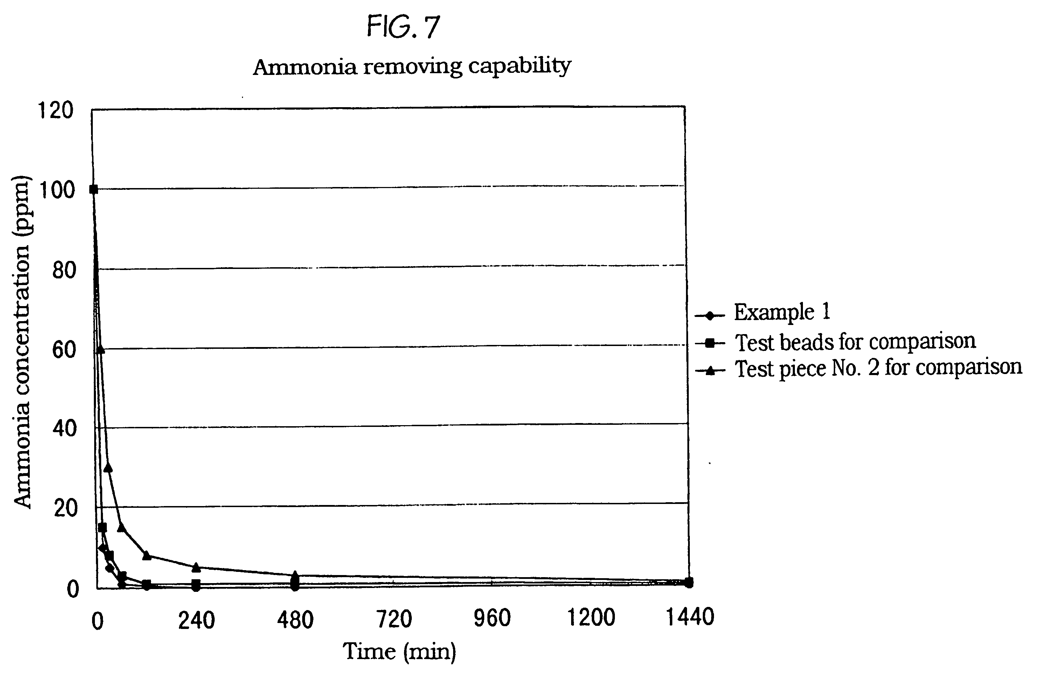

The product obtained in Example 1 was cut out in squares of one side 10 cm×10 cm and used as test pieces. The test piece was placed in a bag (3.0 L content) made of a film having high gas barrier property and the air inside was removed once. A 100 ppm nitrogen dioxide gas (3.0 L) and the test piece was sealed therein and placed at 30 cm under a 15W fluorescent lamp. Similarly, a 100 ppm ammonium gas (3.0 L) and the test piece were sealed in a bag prepared in a similar manner and placed at 30 cm under a 15W fluorescent lamp. As a comparative test beads, zeolite-cellulose bead composite (10 g) was used and, as a comparative test piece No. 2, a test piece having the same shape and obtained by dispersing colloidal silica and titanium dioxide in cement for general building materials was used. The nitrogen dioxide gas concentrations inside were measured with the lapse of time, the results of which are shown in Fig. 6. The ammonia gas concentrations inside were measured with the lapse of time, the results of which are shown in Fig. 7. As shown in Fig. 6 and Fig. 7, the product obtained in Example 1 showed most superior gas removing capability of nitrogen dioxide gas and ammonia gas.

### Production Example 2

Pulp (200 g) was impregnated with an aqueous solution of sodium methasilicate 9 hydrate (190 g/5000 ml) and a mixed aqueous solution (5000 ml) of sodium aluminate (150 g) and sodium hydroxide (330 g) was added, which was followed by immersion at 90°C for 2 hr to give 290 g of zeolite-pulp composite. The zeolite-pulp composite had a zeolite-holding percentage of 38.0 wt%.

### Example 2 (not according to the invention)

The zeolite-pulp composite (100 g) obtained in Production Example 2 was mixed with wood powder (300 g), polyvinyl alcohol, vinyl acetate adhesive (50 g) and water (200 g), and the mixture was placed in a mold and pressurized to give a product (thickness 3.0 cm).

### Experimental Example 2 (not according to the invention)

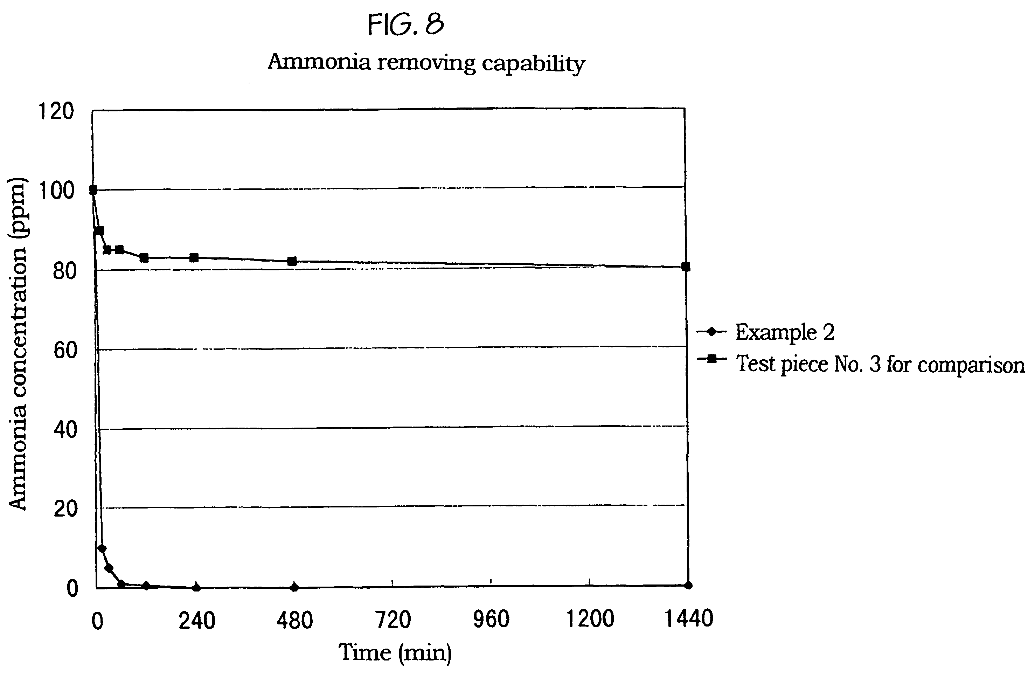

The product obtained in Example 2 was cut out in squares of one side 10 cm × 10 cm and used as test pieces. The test piece was placed in a bag (3.0 L content) made of a film having high gas barrier property and the air inside was removed once. A 100 ppm ammonium gas (3.0 L) was sealed therein. A wood board of the same shape obtained in the same manner as above except that the zeolite-pulp composite was not used was used as comparative test piece No. 3. The ammonia gas concentrations inside were measured with the lapse of time, the results of which are shown in Fig. 8. As shown in Fig. 8, the wood board prepared as comparative test piece scarcely adsorbed ammonia gas, whereas the product obtained in Example 2 showed superior gas removing capability of ammonia gas.

### Example 3 (not according to the invention)

A paper (basic weight 100 g/m², paper width 50 cm) was made from the zeolite-pulp composite obtained in Production Example 2. The paper was impregnated with a copolymer of phenol resin (novolac) and hexamethylene tetramine, which copolymer is used as an undercoating. Excess resin was scraped off to make the total thickness (paper thickness) 100 µm. Ten minutes later, phenol resin cured, whereby a product was obtained.

### Experimental Example 3 (not according to the invention)

The product obtained in Example 3 was cut out in squares of one side 10 cm × 10 cm and used as test pieces. The test piece was immersed in an aqueous solution (100 cm³) of silver nitrate adjusted to 10 ppm and, 30 minutes later, the silver concentration of this aqueous solution was measured and found to be 50 ppb. The test piece noticeably reduced the silver concentration.

### Example 4 (not according to the invention)

Zeolite-pulp composite (100 g) obtained in Production Example 2 was impregnated with an aqueous solution of calcium chloride (0.30 mmol/ 1000 ml) and this zeolite was changed to 5A zeolite having larger pore size. To the 5A zeolite-pulp composite (10.0 g) were added collagen (5.0 g) and calcium phosphate (1.0 g) to give a molded product (5 cm×3 cm×1 cm).

### Experimental Example 4 (not according to the invention)

The product (5 cm×3 cm×1 cm) obtained in Example 4 was immersed in an artificial body fluid (100 cm³) and preserved at 38°C. Thirty days later, the product was taken out and dried, and dry weight was measured. The weight was 15.8 g when first measured but 30 days later, the weight increased to 16.5 g. The surface at the beginning of the test was smooth, but gritty 30 days later due to generation of artificial bone. The calcium and phosphorus concentrations of the artificial body fluid were measured, which were found to be 30% less than the concentrations at the beginning of the test.

### Example 5 (not according to the invention)

Untreated pulp (450 g) was added to the zeolite-pulp composite (200 g) obtained in Production Example 1 and the mixture was placed in a chest. Water (65 L) was added to make the pulp slurry concentration 1 wt% and the mixture was stirred for 1 hour. This mixed pulp slurry was applied to an inclined wire-netting paper making machine (angle of inclination 5°, rate 10 m/min) to make a paper (basic weight 100 g/m², paper width 50 cm).

### Comparative Example 1

Water (20 L) was added to the zeolite-pulp composite (200 g) obtained in Production Example 2 to make the pulp slurry concentration 1% and the mixture was stirred for 1 hour. This mixed pulp slurry was applied to an inclined wire-netting paper making machine (angle of inclination 5°, rate 10 m/min) to make a paper (basic weight 100 g/m², paper width 50 cm).

### Experimental Example 5 (not according to the invention)

The papers obtained in Example 5 and Comparative Example 1 were tested for the compression strength in the CD direction according to JIS P8126, tensile strength in the CD direction according to JIS P8113, tear strength in the CD direction according to JIS P8116, and burst strength in the CD direction according to JIS P8112. In addtion, density was measured according to JIS P8118.

The results are shown in Table 1. In the Table, the unit of the compression strength and tensile strength is kgf, the unit of the burst strength is kgf/cm², the unit of the tear strength is gf and the unit of the density is g/cm³.

**Table 1**

| | Compression strength | Tensile strength | Tear strength | Burst strength |
|---|---|---|---|---|
| Example 5 | 7.0 | 2.2 | 230 | 2.5 |
| Comparative Example 1 | 2.0 | 0.5 | 30 | 1.3 |

The paper of Example 5 was superior to the paper of Comparative Example 1 in every strength test. This is attributable to the stronger hydrogen bond in the paper of Example 5 than in the paper of Comparative Example 1.

### Production Example 3 (not according to the invention)

Pulp (300 g) was impregnated with an aqueous solution of sodium methasilicate 9 hydrate (190 g/5000 ml) and a mixed aqueous solution (5000 ml) of sodium aluminate (150 g) and sodium hydroxide (330 g) was added, which was followed by immersion at 90°C for 2 hr to give a zeolite-pulp composite. The zeolite-pulp composite thus obtained had a zeolite-holding percentage of 30.1 wt%.

This zeolite-pulp composite was applied to an inclined wire-netting paper making machine (angle of inclination 5°, rate 10 m/min) to make a zeolite-holding paper (basic weight 100 g/m², paper width 50 cm).

### Example 6 (not according to the invention)

A rayon nonwoven fabric (basic weight 60 g/m², cloth width 50 cm) prepared by wet method was adhered to the zeolite-holding paper obtained in Production Example 3 using a starch clue as a binder, whereby a laminate was obtained.

### Example 7 (not according to the invention)

The zeolite-holding paper obtained in Production Example 3 was passed through a thermal roll and heated to make the surface temperature 150°C. A nonwoven fabric (thickness 30 µm) made from a polyethylene-polypropylene composite fiber prepared by dry method was continuously brought into contact therewith while spraying an absorbing polymer powder, to give a laminate.

### Example 8 (not according to the invention)

The zeolite-holding paper obtained in Production Example 3 was passed through a thermal roll and heated to make the surface temperature 150°C. This step was done in two series and a low density polyethylene (LDPE film, thickness 40 µm, width 50 cm) was continuously brought into contact with the papers by placing the polyethylene between them, whereby a laminate was obtained, which carried LDPE film between these zeolite-holding papers upon heat-melting.

### Example 9 (not according to the invention)

The zeolite-holding paper obtained in Production Example 3 was passed through thermal roll and heated to make the surface temperature 150°C. This step was done in two series and a polyethylene nonwoven fabric (thickness 30 µm) obtained by dry method was continuously brought into contact with the papers by placing the fabric between them, whereby a laminate was obtained, which carried polyethylene nonwoven fabric between these zeolite-holding papers upon heat-melting.

### Production Example 4

Cellophane (200 g) was impregnated with an aqueous solution of sodium methasilicate 9 hydrate (190 g/5000 ml) and a mixed aqueous solution (5000 ml) of sodium aluminate (150 g) and sodium hydroxide (330 g) was added, which was followed by reaction for 4 hr in a steam generator wherein steam (110°C) was generated to give zeolite-cellophane composite. The zeolite-cellophane composite had a zeolite-holding percentage of 15.3 wt%.

### Example 10

The zeolite-cellophane composite obtained in Production Example 4 was passed through a thermal roll and heated to make the surface temperature 150°C. This step was done in two series and LDPE film (thickness 40 µm, width 50 cm) was continuously brought into contact with the two cellophanes by placing the film between them, whereby a laminate was obtained, which carried LDPE film between the holding cellophane upon heat-melting.

### Example 11

The laminate (15.0 g) obtained in Example 10 was immersed in an aqueous solution (500 ml) of copper sulfate 5 hydrate (2.20 g) for 2 hours, whereby a copper (5.0 mmol)-holding laminate was obtained.

### Example 12

A high strength rayon fiber (100 g) comprising rayon and polypropylene, polypropylene being inserted into the core section of the rayon fiber when regenerating the rayon, and having polypropylene (core diameter 20 µm; fineness 50 µm; average fiber length 20 mm) was impregnated with an aqueous solution of sodium methasilicate 9 hydrate (85 g/2000 ml) and a mixed aqueous solution (2000 ml) of sodium aluminate (80 g) and sodium hydroxide (160 g), which was followed by immersion at 90°C for 2 hr to give a zeolite-rayon composite fiber having the shape shown in Fig. 4. This zeolite-rayon composite fiber was highly strong and zeolite has been present only in the rayon section. The zeolite-rayon composite fiber had a zeolite-holding percentage of 23.5 wt%.

### Example 13

A nonwoven fabric produced from the zeolite-rayon composite fiber obtained in Example 12 by a wet method, and a polyethylene nonwoven fabric prepared by a dry method were heat treated in the same manner as in Example 8, brought into contact with each other and melt-adhered. Then, a sheet made from an active charcoal fiber by a dry method was brought into contact therewith and melt-adhered to give a three-layer laminate.

### Example 14

A foamed polyurethane sheet (thickness 10 mm) was adhered to the laminate obtained in Example 9 via a vinyl acetate resin emulsion adhesive and an aluminum foil (thickness 10 µm) was adhered thereto, whereby a 4-layer laminate shown in Fig. 5 was obtained.

### Example 15 (not according to the invention)

Wall mortar (1 kg) was dissolved in water (1 L) and poured into a mold (25 cm × 25 cm × 5 cm) made of an acrylic plate (thickness 5 mm). When this mortar became half dry, the zeolite-holding paper prepared in Production Example 3 was placed thereon, which was allowed to stand to complete dryness. The mold was removed and colloidal silica containing titanium dioxide dispersed therein was applied to a plane opposite to the plane, to which the zeolite-holding paper had been adhered, to the thickness of 50 µm, whereby a laminate was obtained.

The above-mentioned laminate obtained in Example 6 is superior in surface appearance and a touch and can remove bad smells, so that it is preferably used as a material for wall paper. The laminate obtained in Example 7 is preferably used as a deodorant sheet for paper diapers, sanitary items, and the like. The laminate obtained in Example 8 is preferably used as a freshness retaining sheet for fruits and vegetables. The laminate obtained in Example 9 is preferably used as a gaseous or liquid phase filter, since it has high strength and superior gas permeability by sandwiching a polyethylene nonwoven fabric between two sheets of zeolite-holding paper. The laminates obtained in Examples 12 and 13 are also preferably used as a gaseous or liquid phase filter as in Example 9. In particular, the laminate obtained in Example 13 adsorbs not only polar molecules but also nonpolar molecules, which makes it useful as a gaseous phase filter.

The laminate obtained in Example 10 is preferably used as a cation exchange sheet, and the laminate obtained in Example 11 is preferably used as an antibacterial sheet since it exerts antibacterial property due to copper ion.

The laminate obtained in Example 14 is preferably used as a building panel material, since it is superior in heat insulation, buffering and electromagnetic resistance, adsorbs abnormally smelling gases, and has high strength. The laminate obtained in Example 15 is also preferably used as a building panel material as in Example 14, and exhibits optical catalyst function due to titanium dioxide.

When an inorganic porous zeolite crystals-cellulose composite (A) wherein the cellulose composite contains inorganic porous zeolite crystals in its inner matrix and a function improver (B), function improving fiber (B1) or function improving substrate (B2) are combined, the product, textile, nonwoven fabric, paper and laminate obtained from the composition of the present invention come to have high strength, in addition to the gas adsorption capability, volatile organic solvent removing capability, noncombustibility, heat insulating property, and heavy metal and radioactive element removing capability, that the inorganic porous zeolite crystals-cellulose composite (A) possesses. It is also possible to improve a touch and so that the composition is useful as a material having additional functions. By making an inorganic porous crystal hold a metal, a composition, product, textile, nonwoven fabric, paper and laminate further having antibacterial property and bad smell removing capability can be provided.

## Claims

1. A composition comprising
(A) an inorganic porous zeolite crystals-cellulose composite wherein
(i) the cellulose is natural cellulose which swells with water, and
(ii) a part or the entirety of the inorganic porous zeolite crystals is contained in the' inner matrix of the cellulose, the inner matrix being the inside of a substance constituting the cellulose not including fine pores and lumen present on the surface of the cell wall or in the cell wall of the cellulose, and
(B) a function improver which is a carrier capable of solidifying and being a polyolefin.

2. The composition of claim 1, wherein the inorganic porous crystals hold at least one metal selected from the group consisting of silver, copper, zinc, iron, nickel, cobalt, palladium and platinum.

3. The composition of claim 1, wherein the natural cellulose is at least one member selected from the group consisting of wood powder, pulp, cotton, hemp and kenaf.

4. A product obtainable by solidifying the composition as defined in any of claims 1 to 3.

5. The product of claim 4, which is a laminate comprising a layer made from composite (A) and a function improving substrate (B2) being a polyolefin.

6. The product of claim 4 being selected from a textile, nonwoven fabric or paper comprising a fiber made from composite (A) and a function improving fiber (B1) being a polyolefin.

## Patentansprüche

1. Zusammensetzung, umfassend
(A) einen anorganischen, porösen Zeolithkristall-Cellulose-Verbundstoff, wobei
(i) die Cellulose natürliche Cellulose ist, die mit Wasser quillt, und
(ii) ein Teil der Gesamtheit der anorganischen porösen Zeolithkristalle in der inneren Matrix der Cellulose enthalten ist, wobei es sich bei der inneren Matrix um das Innere einer Substanz handelt, aus der die Cellulose besteht, die keine feinen Poren und kein Lumen, die auf der Oberfläche der Zellwandung oder in der Zellwandung der Cellulose vorhanden sind, umfasst, und
(B) einen Funktionsverbesserer, bei dem es sich um einen Träger handelt, der zur Verfestigung fähig ist und bei dem es sich um ein Polyolefin handelt.

2. Zusammensetzung nach Anspruch 1, wobei die anorganischen, porösen Kristalle wenigstens ein Metall halten, das aus der aus Silber, Kupfer, Zink, Eisen, Nickel, Kobalt, Palladium und Platin bestehenden Gruppe ausgewählt ist.

3. Zusammensetzung nach Anspruch 1, wobei es sich bei der natürlichen Cellulose um wenigstens ein Element handelt, das aus der aus Holzpulver, Zellstoff, Baumwolle, Hanf und Kenaf bestehenden Gruppe ausgewählt ist.

4. Produkt, erhältlich durch das Verfestigen der in einem der Ansprüche 1 bis 3 definierten Zusammensetzung.

5. Produkt nach Anspruch 4, bei dem es sich um ein Laminat handelt, das eine Schicht aus Verbundstoff (A) und ein funktionsverbesserndes Substrat (B2), bei dem es sich um ein Polyolefin handelt, umfasst.

6. Produkt nach Anspruch 4, ausgewählt aus einem Textilerzeugnis, einem Vliesstoff oder einem Papier, das eine Faser aus Verbundstoff (A) und eine funktionsverbessernde Faser (B1), bei der es sich um ein Polyolefin handelt, umfasst.

## Revendications

1. Composition comprenant
(A) un matériau composite de cristaux inorganiques poreux de zéolithe et de cellulose dans lequel
(i) la cellulose est une cellulose naturelle gonflant au contact de l'eau, et
(ii) une partie ou la totalité des cristaux inorganiques poreux de zéolithe est contenue dans la matrice interne de la cellulose, la matrice interne étant l'intérieur d'une substance constituant la cellulose ne comprenant pas les fins pores et lumens présents à la surface de la paroi cellulaire ou dans la paroi cellulaire de la cellulose, et
(B) un améliorateur de fonctions qui est un support capable de se solidifier et qui est une polyoléfine.

2. Composition selon la revendication 1, dans laquelle les cristaux inorganiques poreux contiennent au moins un métal choisi dans le groupe constitué par l'argent, le cuivre, le zinc, le fer, le nickel, le cobalt, le palladium et le platine.

3. Composition selon la revendication 1, dans laquelle la cellulose naturelle est au moins un élément choisi dans le groupe constitué par la poudre de bois, la pâte de cellulose, le coton, le chanvre et le kénaf.

4. Produit pouvant être obtenu par solidification de la composition selon l'une quelconque des revendications 1 à 3.

5. Produit selon la revendication 4, qui est un stratifié comprenant une couche composée du matériau composite (A) et d'un substrat améliorateur de fonctions (B2) qui est une polyoléfine.

6. Produit selon la revendication 4, qui est choisi parmi un textile, un non-tissé ou un papier comprenant une fibre composée du matériau composite (A) et d'une fibre amélioratrice de fonctions (B1) qui est une polyoléfine.
